(19) Europäisches Patentamt
European Patent Office
Office européen des brevets

(11) **EP 2 177 179 B1**

(12) **EUROPEAN PATENT SPECIFICATION**

(45) Date of publication and mention
of the grant of the patent:
**15.06.2011 Bulletin 2011/24**

(51) Int Cl.:
**A61F 2/16** (2006.01)

(21) Application number: **08166689.3**

(22) Date of filing: **15.10.2008**

(54) **Method for modelling an intraocular lens and intraocular lens**

Verfahren zum Modellieren einer Intraokularlinse und Intraokularlinse

Procédé pour le modelage d'une lentille intraoculaire et lentille intraoculaire

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**21.04.2010 Bulletin 2010/16**

(73) Proprietor: **Carl Zeiss Meditec France S.A.S.**
**17053 La Rochelle Cedex 9 (FR)**

(72) Inventors:
• **Gatinel, Damien**
**75019, Paris (FR)**
• **Gerlach, Mario**
**07768, Altenberga (DE)**
• **Buchheister, Jan**
**07751, Jena (DE)**

(74) Representative: **Lechner, Armin Anton**
**Hofstetter, Schurack & Skora**
**Patentanwälte**
**Balanstraße 57**
**81541 München (DE)**

(56) References cited:
WO-A1-2007/128423    WO-A1-2008/094518
WO-A2-2006/108004    US-A- 4 581 033
US-A- 5 108 429    US-A1- 2005 060 032
US-A1- 2007 032 868

• RAWER ET AL: "Imaging quality of intraocular lenses" JOURNAL CATARACT AND REFRACTIVE SURGERY, SURGERY, FAIRFAX, VA, vol. 31, no. 8, 1 August 2005 (2005-08-01), pages 1618-1631, XP005045238 ISSN: 0886-3350
• ANONYMOUS: "ZEMAX: Software For Optical System Design" INTERNET ARTICLE, [Online] 30 April 2007 (2007-04-30), XP002511774 Retrieved from the Internet: URL:http: //web.archive.org/web/20070430071 504/http: //www.zemax.com/index.php?option= com_ content&task=category&sectionid=12&id= 48&Itemid=133> [retrieved on 2009-01-28]

EP 2 177 179 B1

## Description

### Field of the invention

The invention relates to a method for modelling an intraocular lens that provide the human eye with reduced visual defects.

### Background of the invention

The treatment of Cataract, as the worlds most cause for blindness, is a well known process since the ages of the antique Rome (1st and 2nd century AD). Since that ancient time, the complete removal of the opaque human crystalline lens is still the best choice to partially restore visual acuity of the patients. The achieved results are expectedly bad, because of the disregarded refractive contributions of the natural human lens to the visual apparatus, which is not adequately compensated in this situation.

A breakthrough in Cataract surgery appeared to be in 1949 as the English Doctor Harold Ridley successfully implanted the first intraocular lens made of hard PMMA plastics. This lens was capable to compensate for lost optical power of the natural human lens. Since this early time IOLs (intraocular lenses) and surgery techniques were continuously improved. Today Cataract surgery is the far most performed surgery in ophthalmology with more than 2.3 million patients per year in the United States. Approximately another 3 million surgeries add from Europe and Japan. In many cases, however, the optical performance of the IOL may be degraded by inherent corneal aberrations. The human cornea generally exhibits a positive spherical aberration, which is typically offset by a negative spherical aberration of the natural crystalline lens. If this positive spherical aberration of the cornea is not accounted for, it will adversely affect the focusing of light by the combined system of cornea and an implanted IOL.

The optical performance of the pseudophakic human eye, in the understanding of an optical system, is the best if the artificial lens is properly positioned with respect to the visual axis, in a best-focus position with respect to the retinal receptor cells and the total post-op aberrations of the entire optical pathway are reasonably small. By aberrations in this context is meant wavefront aberrations. This is based on the understanding that a converging wavefront must be perfectly spherical to form an ideal point image, i.e. if a perfect image shall be formed on the retinal receptor layer of the eye, the wavefront having passed the optical surfaces of the eye, such as the cornea and a natural or artificial lens, must be perfectly spherical. An aberrated image will be formed if the wavefront deviates from being spherical. By fulfillment of this condition the incoming rays from distant object points form minimum blurred spots at the retina and provide sharp vision in a ray model. The quality of the point image is then degraded by diffraction effects at the aperture stop only and therefore called "diffraction limited".

The correct selection of an IOL in order to match the individual demands of a patient's eye is still difficult and the post operative optical error of the patients depends on four major classes of error contributions. The total postoperative error is a squared sum following the error propagation equation of each group's contribution.

$$\Delta_{total} = \sqrt{\Delta_{Biometry,pre}^2 + \Delta_{Biometry,post}^2 + \Delta_{IOLPower}^2 + \Delta_{Aberration}^2}$$

The first class ($\Delta_{Biometry, pre}$) includes preoperative biometry errors such as keratometry errors ($\Delta K$), ACD (anterior chamber depth)-errors $\Delta ACD$, axial length errors ($\Delta_{AL}$), the accuracy of the IOL power equation used ($\Delta_{equation}$), corneal asphericity errors ($\Delta_Q$) and may provide erroneous input data for the IOL calculation:

$$\Delta_{Biometry,pre} = \sqrt{\Delta_{ACD}^2 + \Delta_K^2 + \Delta_{AL}^2 + \Delta_{formula}^2 + (\Delta_Q^2)}$$

The second group ($\Delta_{Biometry, pre}$) accounts for postoperative biometry changes such as surgical trauma, wound healing processes and capsular symphysis.

$$\Delta_{Biometry, post} = \sqrt{\Delta_{postACD}^2 + \Delta_K^2}$$

The third group ($\Delta_{IOLPower}$) includes the limited accuracy of the IOL labeling power, the IOL power increments defined by the IOL manufacturer, the accuracy of the IOL-constant (e.g. A-constant, Haigis constant, ELP (effective lens position) etc.) and the relative shift of the second principal plane as a function of the IOL design at different optical powers.

$$\Delta_{IOLPower} = \sqrt{\Delta_{Power\,Increments}^2 + \Delta_{IOL\,constant}^2 + \Delta_{Labeling\,accuracy}^2 + \Delta_{SPP}^2}$$

The last group $\Delta_{Aberration}$) includes post optical aberrations such as remaining cylinder, remaining SA (spherical aberration) and a summation of higher order aberrations.

$$\Delta_{Aberration} = \sqrt{\Delta_{Cylinder}^2 + \Delta_{SA}^2 + \Delta_{HOAS}^2}$$

In order to minimize the postoperative optical errors in the aforementioned groups, doctors, researchers and industry found ways to reduce the contribution of the individual error components. The regular use of the Carl Zeiss IOLMaster defines the Gold Standard in biometry measurements and improved clinically significant the accuracy of the preoperative biometry data assessment. Modern IOL calculation formulas are more accurate for non-standard biometries and support the optimization of the IOL-constants on a physician and IOL specific level.

Novel surgical techniques such as micro-incision surgery (MICS) reduce the risk of inducing undesired postoperative corneal astigmatism. Furthermore astigmatism neutral incision techniques and limbal relaxation incisions help to reduce pre-existing corneal astigmatism.

In two particular studies, the development of the components of the eye were examined separately, leading to the conclusion that the optical aberrations of the individual components of younger eyes cancel each other out. It is known that the corneal data can be used together with other ocular parameters to predict the power and the asphericity of an intraocular lens with the purpose of maximizing the optical performances of the future pseudophakic eye. Furthermore, it was also observed that the shape of the cornea in the subjects provides a positive spherical. Studies indicate that the cornea in the subjects provides a positive spherical aberration, which increases slightly with the age. On the other hand, the rotationally symmetric aberration of the anterior corneal surface does not seem to be different between younger and older eye according to further studies.

The deteriorating effect of spherical aberration is mitigated by the use of spherical aberration correcting IOLs. It is well known from the prior art, that spherical aberration compensation in IOLs can be achieved using aspherical surface modifications. Aspherical IOLs can be constructed with symmetrical surface shapes or asymmetrical surface shapes.

One example of such an asymmetrical aspherical IOL is disclosed in US 6,609,793 B2. The anterior surface of this IOL has been designed to be aspherical. The deviations from the spherical base curve are expressed as 6th order polynomial expansion. The IOL design is based on averaged wavefront aberrometry data obtained on a large patient cohort. The objective of the aspherization is to compensate for the positive spherical aberration as induced by the average human cornea. The IOL is designed to provide a certain amount of negative spherical aberration in order to adjust the entire optical apparatus to zero spherical aberration. As viewed from a theoretical perspective this design provides maximum optical performance at the narrowest possible point spread function provided that the corneal spherical aberration is identical or very close to the average amount of the model population used for the design.

A more advanced example of an aspherical IOL providing spherical aberration correction is diclosed in DE 10 2006 021 521 A1. Besides correction of spherical vision errors the surface modifications according to the invention allow the restoration of the optical properties of the natural human lens, as existing prior to extraction. In addition the intentional balancing of the aforesaid anterior and posterior surface modulations provide minimum sensitivity of the optical performance with regards to mechanical positioning disturbances such as decentration and tilt of the IOL that may be induced due to surgery inaccuracy, surgical trauma or capsular bag symphysis. This is achieved by intentional adjustment of the optical aberrations in a way to make them similar to the effects of the natural human crystalline lens.

The optical improvement potential of aspherical IOLs is well known from the prior art. But to get the best benefit of their use, the patient's biometry and corneal topography should be identical or very close to the average amount of the model population used for the initial design. The benefit from one-fits-all approach fails if the particular patient's ocular properties do not match the design conditions. In this case the IOL will have a detoriating effect on optical performance. An extension of this aspect is the impact of accidental reverse orientation of asymmetrical IOLs as compared to the initial design model. Several examples for the influence of implantation orientation and different IOL designs on image quality can be found in the paper of R. Rawer at al, "Imaging quality of intraocular lenses", Journal Cataract Refractive Surgery - Vol 31, August 2005 *and* Spraul, Rawer, Klin. Monatsblätter d. Augenheilkunde 2005; 222:972-976 "Spielt die Orientierung der IOL im Auge eine Rolle*".*

An IOL based on an averaged cornea model will be sufficient for a large number of patients. But unfortunately, there are a significant number of patients, whose cornea won't fit an averaged cornea. The reason for such a deviation from average are manifold, some important reasons are listed next:

A difference to a generalized averaged cornea could just be a difference of averaged cornea shapes between ethnic groups. Also, most of treated corneas due to refractive surgical procedures such as RK (radial keratotomy), LASIK (laser-in-situ-keratomileusis), LASEK (laser-epitheliale-keratomileusis), PRK (photo refractive keratectomy) show typically corneal shapes differing from a generalized averaged cornea. This volume of this group is increasing, because of steeply increasing number of refractive surgical treatments world wide.

Furthermore, very steep and very flat corneas do provide atypical amounts of spherical aberration, which won't be corrected by an aspheric IOL providing nearly perfect correction for the averaged cornea.

It is an object of the invention to provide a method for modelling an intraocular lens that results in at least reduced visual defects of an human eye. It is a further object of the invention to provide a method for modelling an intraocular lens and such an intraocular lens that reduces the relatively low applicability of intraocular lenses well known in the prior art. Further it is an object of the invention to provide a method for modelling an introcular lens and such an intraocular lens that decreases the large number of different intraocular lenses required for correcting known different visual defects of human eyes.

## Description of the invention

The invention relates to a method for modelling a single intraocular lens that provide the human eye with reduced visual defects. The method comprises modelling the shape of at least one surface of an intraocular lens and/or modelling the interior of said intraocular lens with respect to their refraction effect in such a way that with a first orientation within a human eye the intraocular lens at least partially corrects a first visual defect of a human eye and with a second orientation within the human eye the intraocular lens at least partially corrects a second visual defect of a human eye.

Therefore a method is given, which enables a very effective and efficient modelling process. Especially wiht the focus on modelling in a dual way in only one designing process with respect to correcting more visual defects with one lens. Therefore with only one lens it is possible to correct at least two different visual defects depending on the orientation of the lens in the human eye. The large number of different lens wich are able to correct only one visual defect is reduced tremendous. Further the applicability of an intracoular lens is inscreased, because the calculated steps taken allow a specific and deliberate correction of at least two visual defects with only one lens. The method allows modelling the lens such that at least two different visual errors are at least corrected in a deliberate and conscious way.

Orientation of the lens means which of the both surfaces, front surface or back surface, of the lens is turned towards the cornea, when the lens is placed in a human eye. Further the lens can have different positions in the human eye, especially depending on angulation and the orientation of angulation of haptic parts of the lens. Therefore placement of the lens within the human eye is especially defined with its orientation and with its position. Preferably the first orientation is the inverted or reversed second orientation.

Especially modelling method comprising surface shape modifications that differ from perfect spherical geometries of the optical surfaces. Besides correction of spherical refractive vision errors, these surface modifications allow the restoration of the optical properties of the healthy natural human lens. In order to match the implant with the particular optical properties of the patient's optical system, the IOL is designed to exhibit two distinct, but well defined, optical properties depending on its direction of implantation in the patient's eye. In addition or instead of said surface shape modification modelling the interior of said intraocular lens, especially modelling refraction effect of the interior leads to the same advantages.

Preferably the method comprising the step of coupling changing the shape of the lens with respect to correct a first visual defect of an eye to changing the shape of the lens with respect to correct a second visual defect of an eye. Especially the mode respectively the way of coupling shape-changing is depending on the specific visual defects to be corrected with the lens.

Especially the modelling method comprising the step of changing a second surface of the intraocular lens defending on the changing of a first surface of the lens with respect to correct at least two different visual defects of eyes depending on the orientation of the lens within an eye. The same applies to changing the interior of the lens.

Modelling the interior of the intraocular lens concerns especially variation of the refractive power of the inner part of the lens. Preferably a continuous variation of the refractive power of the medium respectively the material of the lens is done.

At least one mathematical model of a human eye is used as a basis for modelling the intraocular lens by way of inserting the intraocular lens instead of the human crystalline into said mathematical model to correct either said first or said second visual defect, depending at least on the orientation of the intraocular lens within the human eye.

In a preferred embodiment of the invention two different specifications of a mathematical model are used as a basis for modelling the intraocular lens for the correction of two different visual defects, wherein said first specification characterizes said first visual defect and said second specification characterizes said second visual defect. Therefore a mathematical model is chosen in its basic state. To characterize a special visual defect this basic state of a mathematical model is changed in an specific way, such that this specific visual defect is also descripted by a mathematical model. To change the mathematical model with respect to characterize a specific visual defect one or more parameters are changed. Therefore in the modelling process a mathematical model is preferably used in a changed state in comparison with its basic state.

Possible mathematical models are for example the Liou Brennan eye model, the Navarro eye model, the Dubbelman eye model or the Gullstrand eye model. Each of these models have a specifc and individual basic state.

Said special models are only possibilities and other eye models could also be the base for the method for modelling an intraocular lens of course. Depending of the at least two specific visual defects to be corrected with the modelled intraocular lens the therefore most adequate models are chosen. Many combinations of models can be done, depending on the given situation for modelling the lens with respect to the visual defects to be corrected. For example the Liou Brennan model or specifications thereof enable characterizing non rotation-symmetrical visual defects. For example the Navarro model or specifications thereof enable characterizing rotation-symmetrical visual defects, like defocusing or spherical aberration.

Preferably two different mathematical models are used as a basis for modelling the intraocular lens in two different ways, wherein said first mathematical model characterizes said first visual defect and said second mathematical model characterizes said second visual defect. It is possible to chose one of the above mentioned models and create different variations for characterizing respectively defining best the specific visual defects to be corrected with the lens. This embodiment enables modelling with one basic eye model, where some general values and parameters characterizing the eye model are based for both specification and therefore a general data base for both visual defects is provided. On the other side other values and parameters characterizing the eye model are specifically changed to create the two specifications of the eye model. Therefore for modelling the lens and to correct at least both visual defects with the modelled intraocular lens a common data base is based. This enables very accurate modelling with respect to very exact corrections of both visual defects. Further the modelling efforts are reduced.

In a preferred embodiment said modelling generates an intraocular lens which functions as an aberration-correcting lens with a first orientation within the human eye.

Preferably said modelling generates an intraocular lens which functions as an aberration-corrected lens with a second orientation within the human eye.

Said at least one mathematical model is preferably characterized by an aspherical shape of the cornea and/or a gradient index of the refractive medium of said model eye and/or an aspherical shape of the natural human crystalline and/or the length of the bulbus. These are very characteristic parameters with respect to describe a human eye. So these parameters enable a very accurate characterization of for example a specification of a mathematical model. Exact definitions of such model specifications are therefore possible, which further leads to exact modelling of the lens and very good results for corrections for not only one of the visual defects but for both visual defects to be corrected with the single lens.

In a preferred embodiment modelling including defining the connections respectively linking of at least one parameter of each mathematical model or of each specification of one mathematical model with each other. Especially the mode of linking of the parameters and/or which parameters are linked is depending on the at least two visual defects to be corrected with the lens. The parameters characterizing the mathematical models or the specifications of a mathematical model used as a basis for modelling and in which upon changing the at least one parameter of one model or one specification one parameter of the other model or the other specification of a model is changed in such a way that the shape of the surfaces of the intraocular lens and/or its interior with respect to its refraction effect is changed for correction of the first and the second visual defect.

Preferably at least one of said surfaces of the intraocular lens is designed as an aspherical surface. Also both outer surfaces of the intraocular lens can be designed as aspherical surfaces.

Preferably modelling the intraocular lens comprising an iterative process. Therefore during the modelling process the momentary modelled state of the intraocular lens is checked to the effect if correction of the visual defects is possible with sufficient size. This is done for both visual defects to be corrected with the lens. If the result of correction size is not sufficient for at least one of the visual defects the modelling process is continued. Therefore the momentary modelled result of the lens is specifically changed. Especially changing of the momentary embodiment of the intraocular lens is depending of the momentary embodiment itself and/or the degree of failure of the achieved result of correction of the visual defects in comparison to the final degree of the correction which should be achieved at the end of the modelling process.

Especially in only one file of a design programme or a modelling programme two different mathematical eye models are preseted by constructing so called configurations. Therefore different forms of an eye are configured, where each configuration characterize a situation of a human eye with a specific visual defect. A visual defect therefore can be arise in fact of disease of the eye and/or as a result of a surgical treatment. For example a first configuration characterize a typical forming of a cornea after a refractive surgery of the eye. Another or second configuration characterize a typical european cornea or a typical asian cornea. These are only possible configurations. There is a large number of other configurations.

In a further step of the iterative process the respective parameters or other values characterizing the intraocular lens to be modelled are chosen for each configuration. Then these parameters are connected respectively linked together. The linking of the parameters is done in such a way that after starting the modelling procedure at least one parameter of the second configuration is changed if at least one parameter of the first configuration is changed. Therefore for example if changing a parameter of the first configuration results in changing the shape of a first surface of the intraocular lens at least one parameter of the other configuration is changed automatically in fact of the connection of the parameters in such a way that the shape of the second surface of the intraocular lens is changed.

Examples for parameters are the radius of the cornea and/or the conical constant and/or coefficients of a polynomial asphere and/or the refractive power and/or the spherical aberration and/or the local curvature and/or the distribution of the refractive power. This is not a final enumeration of possible parameters. Of course many more parameters can be based for a model and can be changed for modelling the intraocular lens in the best way with respect to the given specific situation.

In a further preferable step of the method within the iterative process for modelling the intraocular lens the lens has a first or respectively anterior surface and has a second or respectively posterior surface. It is emphasized that the preceding definition of first, second respectively anterior and posterior surface is only one examplary possibility. In a preferred embodiment if at least one parameter of the first configuration is changed the first surface of the lens to be modelled is changed in its shape in a specific way, and because of the connection of parameters of the two different configurations automatically at least one parameter of the second configuration is changed and as a result thereof the second surface of the lens is changed in its shape in a specific way. Therefore in a preferred embodiment if the first surface, which for example is defined as the front respectively anterior surface of the intraocular lens in said first configuration and is the posterior surface of the second configuration, is changed in a specific way, the second surface, which is then the back respectively posterior surface of the intraocular lens in said first configuration and the front respectively anterior surface of the second configuration, is changed in a specific way. This because of the two different visual defects to be corrected with the one single modelled lens depending on the position and the orientation of the lens in the eye. To correct a first visual defect of a first human eye the lens is inserted in this eye with a first orientation. To correct a second visual defect of a second human eye the lens is inserted in this second eye with a second orientation.

In a preferred further step of the iterative process a defined merit function presets the aims to be achieved with the modelling process. Therefore definitions of the objective sizes and weigthing of the objective sizes are predetermined depending of the specific modelling situation. The specific modelling situation depends prior of the chosen mathematical models and/or the specific visual defects to be corrected.

In a preferred step of the iterative process one or more parameters were chosen as variables and the iterative optimization process is started. Preferably this is done for only one configuration. Because of the preferred step of the method comprising connecting of at least some of the parameters of the different configurations this leads to an optimization process for both configurations with respect to correcting both visual defects at least partially.

During the iterative process a preferred step of the method comprising evaluating the merit function, especially evaluating the improvement of the merit function. If this is not fulfilled in an adequate result respectively in an acceptable result, the at least one parameter chosen to be changed is then changed at least once more and/or at least one other parameter is changed. This scenario is repeated as long as an acceptable result is achieved with respect to the at least both visual defects to be corrected with the modelled intraocular lens depending on the position with a specific orientation in the eye. An acceptable result can be defined in a variety of performance targets. Especially tolerance intervals of values of optimiziation parameters can be defined and if the values and the result of the iterative process are included in an interval the process is stopped and the optimization goals are fulfilled.

The order of the steps of the iterative process can vary. Depending on a given and specific modelling situation the process can be adapted and therefore it is very flexible. Checking separate modelling steps and the whole procedure is more flexible and checking temporary results of a modelled lens is easier and can be done faster.

Preferably the connection of the parameters of the two different configurations is defined in such a way that if the specific parameter in the first configuration is changed the corresponding repectively the equal parameter of the second configuration is changed as well.

In a special preferred embodiment at least one of said surfaces of said intraocular lens and/or said interior of said intraocular lens with respect to their refraction effect are configured in such a way that said first and said second visual defect, respectively, are corrected each at a Strehl ratio of at least 0.5, especially of at least 0.8, especially of at least 0.95, relative to the mathematical model or the specification of a mathematical model used as a basis. Both visual defetcs can be corrected with the only one modelled lens in a way very near to the theoretical diffraction limit.

Specific parameters of said mathematical models or said specifications of one mathematical model used as a basis for characterizing the visual defects to be corrected by the intraocular lens are changed. Preferably parameters of the cornea are changed. Preferably the radius of the cornea and/or the curvature of the cornea and/or the conical constant and/or at least one coefficient of a polynomial asphere for characterizing a specific visual defect are changed.

Possible visual defects are for example a so called european cornea or a so called asian cornea. These embodiments can be described with characteristic parameters like the radius, the curvature, the conical constant. For example the European cornea can be characterized by values for the radius between 7,70 mm and 7,85 mm and values for the conical constant between -0,1700 and -0,1900. The Asian cornea can be characterized ba values for the radius between 7,56 mm and 7,69 mm and values for the conical constant between -0,1700 and -0,1800 for example. It is emphasized that this specific values and intervals are only examples. Of course also other values or intervals with other limit values are possible for characterizing said specific cornea geometries.

Further visual defects are for example typical biometry of typical post refractive surgery treated eyes. For example typical spherical aberration of post refractive surgery treated myopic eye or hyperopic eyes. A myopic eye can be characterized by a value of radius of the cornea between 8,1 mm and 8,3 mm and a value of the conical constant between +0,4900 and +0,5100 for example. The hyperopic eye can be characterized by a value of radius of the cornea between 7,4 mm and 7,6 mm and values for the conical constant between -0,4900 and -0,5100 for example. Of course other values or intervals with other limit values are possible.

Further visual defects are for example steep corneas or flat corneas. Very steep corneas can be characterized by a value of radius between 7,3 mm and 7,5 mm and a value of conical constant between -0,1700 and -0,1900 for example. Very flat corneas can be characterized by a value of radius between 8,3 mm and 8,5 mm and a value of conical constant between -0,1700 and -0,1900 for example. Of course these values are only examples an there is also the possibility of other values or limit values for the intervals characterizing these specific cornea shapes.

Especially it is the intention of the method modelling the intraocular lens in such a way that aberrations, especially spherical aberrations , which are caused by different cornea geometries are at least partially corrected, especially with a strehl ratio more than 0.8, especially more than 0.95, for both orientations of the lens.

In a preferred embodiment the method comprising designing an intraocular lens capable of adjusting the aberrations of the eye in order to provide optimal vision correction to patients consisting of the following elements:

- mathematical model eye that describes the optical setup and performance of the human eye, comprising at least one aspherical corneal surface, a gradient index and/or aspherical crystalline lens model;

- Optical modelling of an aspherical lens shape that replaces the natural human crystalline in the eye that corrects for spherical vision errors while keeping specific optical properties of the human lens;

- Optical modelling of said aspherical lens in order to solve two different correction tasks given by two different said mathematical model eyes, depending on the orientation of said aspherical lens.

It is preferred that the anterior surface of the lens has a spherical shape and the posterior surface has an aspherical shape. In a further preferred embodiment the anterior surface of the lens has an aspherical shape and the posterior surface has a spherical shape. It is also possible that both surfaces of the lens are of aspherical shape.

In a preferred embodiment a visual axis of the mathematical model eye is tilted with respect to the axis of symmetry 'optical axis' of the eye and a decentered iris represents a decentered entrance pupil.

A mathematical model describes preferably the statistics of potential lens misalignments and positioning errors induced by surgery or wound healing processes. Further it is preferred that calculation of optical performance and resulting aberrations employs said mathematical eye model convoluted with the statistical model for lens displacements. A further preferred embodiment comprising designing the lens in order to make the pseudophakic eye to have the same amount of spherical aberrations and the same level of image quality than the phakic model eye in function of the pupil diameter at least for one of the said orientations.

Further the radial distribution of refractive optical power is preferably divided in at least three functional zones that account for photopic vision and/or mesopic vision. The optical optimization of the aspherical shape is preferably performed in order to minimize sensitivity of the optical performance parameters with respect to potential lens tilt induced by surgery effects or capsular bag symphysis. The optical optimization of the aspherical shape is preferably performed in order to minimize sensitivity of the optical performance parameters with respect to potential lens decentration induced by surgery effects or wound healing processes.

In a preferred embodiment the optical optimization of the aspherical shape is performed in order to minimize sensitivity of the optical performance parameters with respect to potential lens decentration and potential lens tilt induced by surgery effects or wound healing processes

It is preferred that modelling and optimization of the lens shape includes selecting the radii of base curvature of the anterior and posterior surfaces as well as the central thickness, the edge thickness and the refractive index.

The amount of spherical aberration of the artificial lens is preferably maintained at the level as the one of the natural human lens is maintained over a broad range of pupil diameters.

The modified lens shape is preferably defined in terms of linear combination of polynomials.

The modified lens shape is preferably defined by the equation:

$$z = \frac{cr^2}{1 + \sqrt{1 - (1+Q)c^2 r^2}} + k_2 r^2 + k_4 r^4 + k_6 r^6 + k_8 r^8$$

with

$$c = r_{curv}^{-1} \quad \text{(Curvature = 1 / base radius of curvature)}$$

r = independent variable, radius about optical axis
Q = conic constant
$k_n$ = polynomial coefficient of order n

Therefore the constant Q is preferably 0. The constant can also be a value within the interval between -1 <= Q <= 0. Preferably $k_2$ is 0. It is also possible that $k_n$ for $n \geq 2$ are equal to 0. Preferably all coefficients $k_n$ for $n > 8$ are equal to 0.

The modified lens shape is preferably defined in terms of linear combination of polynomials. Especially the modified lens shape is defined by splines. The modified lens shape is preferably piecewise defined by linear combinations of polynomials. Preferably the optical performance is defined as MTF (modulation transfer function) contrast. Therefore the optical performance is preferably defined as Strehl ratio. It is also possible that the optical performance is defined as wavefront error. Further optical performance is preferably defined in terms of point spread functions and encircled energy.

In a preferred embodiment the aberrations of the entire optical train of the human eye are expressed in linear combinations of Zernike polynomials.

It is also possible that the aberrations of the entire optical train of the human eye are preferably expressed in linear combinations of Seidel polynomials. It is a further possibility that the aberrations of the entire optical train of the human eye are expressed in terms of Fourier decomposition of the OPD / wavefront.

Preferably the intraocular lens is made of soft and transparent biomaterial. The soft material can be preferably a hydrophilic material such as hydrophilic acrylic polymer or copolymer. Further the soft material can be a hydrophobic material such as hydrophobic acrylic or silicon. A further possibility comprising manufacturing the intraocular lens of hard material such as polymethylmethacrylathe also know as PMMA.

An intraocular lens comprises a first surface and a second surface and an interior, wherein said surfaces and/or said interior of said intraocular lens with respect to their refraction effect are configured such that with a first orientation of the intraocular lens within a human eye a first visual defect is at least partially correctable and with a second orientation of the intraocular lens within a human eye a second visual defect is at least partially correctable. Therefore with only one lens it is possible to correct at least two different visual errors depending on the orientation of the lens in a human eye. The large number of different lenses which are able to correct only one visual effect is reduced tremendous. Further the applicability of an intracoular lens is increased, because the calculated steps taken allow a specific and deliberate correction of at least two visual defects with only one lens.

Said introcular lens is preferably configured to function as an aberration-correcting lens in at least said first orientation within a human eye. In a further preferred embodiment said intraocular lens is configured to function as an aberration-corrected lens in at least said second orientation within a human eye. It is emphasized that the declaration of first an second position and first and second orientation with respect to the functions as aberration-correcting and aberration-corrected is only an example. Of course this general declaration can be defined in other ways as well, especially in a reversed declaration.

Preferably at least one of said surfaces of said lens is of aspherical design. Also both surfaces can be of aspherical design, especially of two different aspherical designs. It is possible that the shapes of both surfaces are of spherical design.

Both visual defects can be spherical aberrations.

Said intraocular lens preferably comprising a haptic part, which is preferably tilted in relation to the vertical axis.

Said intraocular lens preferably comprising a first value of a lens constant, in particular the A constant, for said first position with a first orientation within the human eye, and a second value of the lens constant, in particular the A constant, for said second position with a second orientation in the human eye.

Said intraocular lens preferably comprises a first characterizing marker for the first position with a first orientation within the human eye and a second marker for said second position with a second orientation within the human eye.

Said intraocular lens preferably have best visual performance for the average european cornea using one orientation and, using the reversed orientation, best visual performance for an averaged asian cornea is given.

Said intraocular lens is preferably manufactured for typical biometry of eyes after refractive surgery. The first orientation of the lens is thereore preferably used to correct spherical aberration of an averaged eye model of natural untreated eye. The second lens orientation preferably corrects the typical spherical aberration of post refractive surgery treated myopic eyes. The biometry characterizes the physiological quantities, especially the eye models for numerous patient groups to be found.

Preferably said intraocular lens is corrected for typical biometry of eyes after refractive surgery. The first orientation of the lens is used to correct spherical aberration of an averaged eye model of natural untreated eye. The second lens orientation corrects the typical spherical aberration of post refractive surgery treated hyperopic eyes.

Said intraocular lens is manufactured in such a way that it corrects eyes with biometry of typical post refractive surgery treated eyes for myopic and hyperopic correction due to change the orientation.

Preferably said intraocular lens corrects eyes with different radii of the cornea. Preferably first orientation is used to correct very steep cornea shapes and the other orientation is used to correct very flat cornea shapes.

Said intraocular lens preferably comprising significantly reduced sensitivity of the optical performance (MTF) with respect to decentration for one or both correction tasks of the IOL. Said intraocular lens preferably comprising significantly reduced sensitivity of the optical performance (MTF) with respect to tilt for one or both correction tasks of the IOL.

Said intraocular lens is preferably modeled in order to make the pseudophakic eye to have the same amount of spherical aberrations and the same level of image quality than the phakic model eye in function of the pupil diameter for one or both correction tasks of the IOL.

Said intraocular lens is preferably at least partially of soft material, wherein the soft material can be a hydrophilic material such as hydrophilic acrylic polymer or copolymer. The soft material can also be a hydrophobic material such as hydrophobic acrylic or silicon. The intraocular lens can be made of hard material such as polymethylmethacrylathe also know as PMMA as well.

The intraocular lens preferably comprising haptic parts, especially purposefully angulated haptics between the lens equatorial plane and the haptics plane to compensate for principal plane shifts between the directions of implantation. Especially the angle is between 0° <= angle <= 10°.

The angulation of these haptic parts enables pressing on the intraocular lens to the crystalline humour of the eye. Further the variation of the principal plane of the lens can be compensated depending on the angulation of the haptic parts.

Preferably the invention shall provide a new aspherical IOL design approach to overcome the disadvantages of the prior art and shall provide significantly improved perceivable optical performance to patients who need an IOL implant.

A lens container has a dual labeling according to its purpose with respect of an intraocular lens manufactured in such a way that depending at least on its orientation in the human eye different visual defects are corrected. This enables the very safe storage and shipping of the lens on the one side and the easy recognizability of the special emodiment of the lens with respect of the special visual defects to be corrected with on the other side.

Preferably the lens container indicates the correction feature, the corresponding A-constant and a patient application criteria such as for example corneal asphericity, Q-value, keratometry readings, corneal spherical aberration, corneal topography etc.

A lens container preferably indicates the correction feature and a pictogram indicating the orientation of the IOL.

An implantation system has a dual purpose intraocular lens manufactured in such a way that depending on its orientation in the human eye different visual defects are corrected.

The lens preferably is provided with markers that indicate the orientation of the IOL after implantation.

The implantation system is preferably a single-use implantation system, especially a so called pre-loaded system.

Said implantation system preferably comprising a suitable IOL container, cassette or cartridge which can be inserted for implantation. The insertion direction of the cartridge is preferably orientated with respect to the markers on the cartridge or the injector body.

A further aspect of the invention relates to a method for manufacturing an intraocular lens comprising the steps of modelling the intraocular lens by the above explained inventive method or a preferred embodiment thereof and manufacturing said intraocular lens on the basis of the modelled intraocular lens.

A method for correcting visual defects of a human eye comprises the steps of removing the natural human crystalline, modelling an intraocular lens with an inventive method explained above or a preferred embodiment thereof, wherein one of said orientation of the intraocular lens within the human eye corrects the specific visual defect of the human eye at least partially; manufacturing the modelled intraocular lens on the basis of the modelled lens; and implanting said intraocular lens in the human eye in the specific position and with the specific orientation for correction of the specific visual defect to be corrected. This enables a surgical treatment with minimal stress for the patient and the treated human eye. Further a surgical treatment is proposed which avoids side effects better than treatments without this special multifunctional intraocular lens proposed in this invention.

A further aspect of the invention relates to a computer programme product comprising a programme part which is written in a specific programming language such that said programme part alone or taken in combination with at least one further programme part of said computer programme enables modelling an intraocular lens, especially the surfaces of said intraocular lens and/or the interior of said intraocular lens, in its refractive function such that a first visual defect of a human eye is at least partially corrected with a first orientation of the intraocular lens within a human eye, and a second visual defect of a human eye is at least partially corrected with a second orientation of the intraocular lens within a human eye.

Preferred embodiments of the method for modelling an intraocular lens are preferred embodiments of the computer programme product.

**Description of the drawings**

Embodiments of the invention will be described in conjunction with the drawings:

Fig. 1      a schematic drawing of the Liou Brennan eye model;

Fig. 2      an embodiment of the intraocular lens;

Fig. 3      a sectional drawing of an eye with a first position with a first orientation of the intraocular lens in the eye;

Fig. 4      a front view of the first orientation of the introcular lens;

Fig. 5      a sectional drawing of an eye with a sec- ond position with a second orientation of the intraocular lens in the eye;

Fig. 6      a front view of the second orientation of the intraocular lens;

Fig. 7      a sectional drawing of a further embodi- ment of an intraocular lens;

Fig. 8      a sectional drawing of an eye including the inserted intraocular lens of Fig. 7 in a first position with a first orienation;

Fig. 9      a sectional drawing of an eye including the inserted intraocular lens of Fig. 7 in a second position with a second orienta- tion;

Fig. 10      a front view of an embodiment of an in- traocular lens;

Fig. 11      a front view of a further embodiment of an intraocular lens;

Fig. 12      an embodiment of an implanting system for implanting an intraocualr lens in an eye;

Fig. 13      an embodiment of a lens container;

Fig. 14      a diagram where the modulus of the OTF de- pending on the spatial frequeny in cycles is shown for a first visual defect to be corrected with a modelled intraocular lens;

Fig. 15    a diagram where the modulus of the OTF de- pending on the spatial frequeny in cycles is shown for a second visual defect to be corrected with a modelled intraocular lens;

Fig. 16    a diagram where the modulus of the OTF de- pending on the spatial frequeny in cycles is shown for a third visual defect to be corrected with a modelled intraocular lens;

Fig. 17    a diagram where the modulus of the OTF de- pending on the spatial frequeny in cycles is shown for a fourth visual defect to be corrected with a modelled intraocular lens;

Fig. 18    a diagram where the modulus of the OTF de- pending on the spatial frequeny in cycles is shown for a fifth visual defect to be corrected with a modelled intraocular lens;

Fig. 19    a diagram where the modulus of the OTF de- pending on the spatial frequeny in cycles is shown for a sixth visual defect to be corrected with a modelled intraocular lens;

Fig. 20    a diagram where the modulus of the OTF de- pending on the spatial frequeny in cycles is shown for a seventh visual defect to be corrected with a modelled intraocular lens;

Fig.21    a diagram where the modulus of the OTF de- pending on the spatial frequeny in cycles is shown for a eighth visual defect to be corrected with a modelled intraocular lens;

Fig. 22    an exemplary first table with design data for modelling an intraocular lens able to correct two different visual defects de- pending on its placement in the eye; and

Fig. 23    an exemplary table with aberration data based on the data in the first table in Fig. 22 for modelling an intraocular lens able to correct two different visual de- fects depending on its placement in the eye.

**Detailed description of preferred embodiments of the invention**

The image formation in natural human eye is accomplished by the conjunction of the ocular media and interfaces. The main contribution in refractive power (-75%) is provided by the cornea which is the first air/media interface of the human eye. Rays emitted by distant object points enter the cornea almost parallel with respect to the optical axis. The refraction of the cornea bends the rays towards the optical axis into a converging bundle. This bundle of rays passes the anterior chamber and enters the human crystalline lens. If no crystalline is in place the rays would converge to singular diffraction limited small spot at a distance of the inverse corneal power. The spot size is determined by the diffraction effects at the edges of the entrance pupil and the wavelength.
Unfortunately, the optical system is not a perfect world but it has developed over ages and optimized itself. The slight aspherical shape of the cornea acts in conjunction with the non-linear Snell's Law of refraction and does not allow that all rays emitted from a distant point source converge at a singular spot. It appears that rays from the outer portions of the pupil hit the optical axis at a smaller distance than the axial rays do. This effect is called spherical aberration (SA) and is related to a sign. If the pupil edge rays hit the optical axis before the axial rays do, the SA is considered to be 'positive'. Positive spherical lenses show this behavior. If the pupil edge rays hit the optical axis at a more distant point on the optical axis than the axial rays the SA is considered to be 'negative'. This behavior is found on plano- parallel glass plates or negative lenses.
Since the edge rays of the cornea hit the optical axis before the axial rays do it adds positive SA to the optical system. This effect prevents the formation of infinitely sharp macular images. Instead there is plenty of light diffusion in blurred spots. The evolution of the human eye accounts for that by developing a highly complex crystalline lens design. The crystalline lens contributes the missing 25% of refractive power to the optical system in order to adjust the focal length exactly to the available axial length of the human eye. In addition it allows accommodating for different viewing distances by internal adjustment of the refractive lens power. Beyond these obvious facts, the crystalline lens acts as an optical correction means of the human eye that compensates for optical errors as introduced by the cornea. In terms of avoiding excessive spot blurring induced by corneal positive SA, the crystalline provides a well adjusted amount of negative SA that almost perfectly compensates for the amount induced by the cornea. The optical performance of this joint optical system is significantly better than of its single components. This inherent compensation mechanism is even working for different viewing distances and pupil diameters due to different lighting conditions.
The main objective of human eye evolution was not to optimize the theoretical optical performance of the eye such as currently widely announced in terms of point spread functions or Strehl ratios. However, the optical apparatus should provide optical performance that perfectly matches the requirements of the cone and rod structure of the retina, their local density functions and color perception properties. Cone and rods mosaic permit only to see images with maximal spatial frequency of 75 cpd, higher spatial frequency can produce aliasing and distort the perceived image. The optical properties of the visual apparatus, the retinal configuration and the physiological processing of the visual information in the visual cortex determine finally the perceivable visual acuity of the patients. This teaches the main objectives of a novel intraocular lens. The IOL has to restore optical power and aberration characteristics of the natural human lens in order to support the neuro-visual optical system for best perceivable visual performance.

In order to provide a design environment for an IOL a particular theoretical eye model needs to be applied. Many basisc states of them are well known from the literature. For example the basic state of the Gullstrand model, the Navarro model, the Dubbelmann model and so on.

All the referred basic states of theoretical eye models, as well as the majority of published eye models rely on simplified ocular configurations of the human eye. The cornea is reduced to a single surface element and the visual axis is assumed to match exactly the axis of symmetry of the eye. These reduced models try to mimic the optical system and aberrations of the human visual apparatus by use of single faced cornea models that apply some degree of asphericity in order to reflect the practically measurable performance. The authors proved that the referred eye models comply with measured results under given assumptions. However, these eye models disregard the specifics of the anatomy of human eye more or less systematic. The most comprehensive eye model currently available in literature was described by Liou and Brennan.

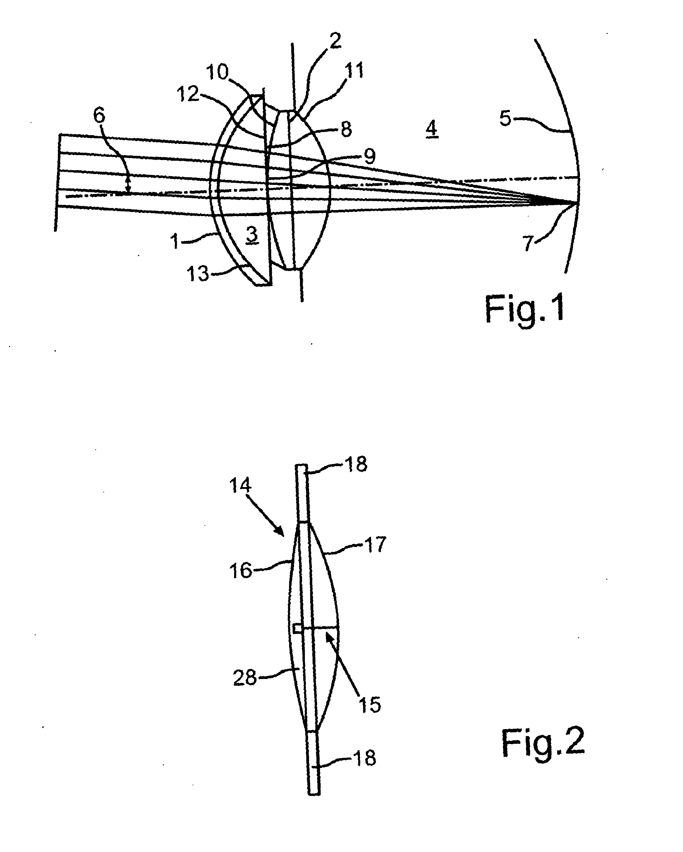

The Liou-Brennan eye, as depicted in Fig. 1, represents the ocular anatomy very closely and preserves the optical properties and aberration characteristics of the human eye. This eye model comprises an aspherical cornea with anterior interface 1 and posterior interface 2, as well as an aspherical gradient-index lens model. The anterior chamber is referred as 3, the vitreous body as 4, the retina as 5. It takes into account that the visual axis is tilted by 5° with respect to the axis of symmetry 6 of the eye to focus in the macular region 7 for the majority of the population. In addition the pupil 8 is slightly decentered by 0.5 mm in nasal direction 9 for the majority of the population. The amount of spherical aberration is balanced by an aspheric cornea that introduces positive spherical aberration. An aspherical natural lens model, comprising two gradient index components 2, 10, 11, provides negative SA to compensate for the corneal contribution. Further the iris plane 12 and the posterior corneal surface 13 are shown in Fig.1. In total the optical train provides a little positive spherical that is equivalent to measured data and helps to increase the depth of focus. In contrary to other model eyes the Liou-Brennan eye consequentially is not rotationally symmetric.

In an embodiment of the development of an intraocular lens involves an eye model that is based on the Liou-Brennan eye model as described by the following surface listing:

| Surf | Comment | Radius | Thickness | Glass | Diameter | Conic |
|---|---|---|---|---|---|---|
| OBJ | - | Infinity | Infinity | - | 0 | 0 |
| 1 | - | Infinity | 1 | - | 4.495 | 0 |
| 2 | - | 0 | - | - | | |
| 3 | CORNEA_FRONT | 7.77 | 0.5 | CORNEA_LB | 12 | -0.18 |
| 4 | CORNEA_BACK | 6.4 | 3.16 | WATER_LB | 12 | -0.6 |
| 5 | PUPIL_DEC_1 | - | 0 | - | - | |
| STO | PUPIL | Infinity | 0 | WATER_LB | 4 | 0 |
| 7 | PUPIL_DEC_2 | - | 0 | - | - | |
| 8 | LENS_FRONT | 12.4 | 1.59 | - | 10 | -0.94 |
| 9 | LEMS_CENTER | Infinity | 2.43 | - | 10 | 0 |
| 10 | LENS_BACK | -8.1 | 16.27 | WATER_LB | 10 | 0.96 |
| IMA | RETINA | -18 | | WATER_LB | 20 | 0 |

A preferred embodiment of the invention is based on specific geometry and/or shape modifications of the basic state of the Liou-Brennan eye model that apply to the anterior or posterior surface or both of the new modelled intraocular lens. Therefore two different specifications of one mathematical model, namely the Liou- Brennan model, are based in the modelling process. The modified IOL surfaces comprise of rotational symmetric deviations from a spherical shape. This procedure is commonly understood as aspherization of optical surfaces.

The direction of implantation of the IOL will impact the optical performance of an IOL in the aphakic eye. The majority of aspherical lenses will cause a degradation of optical image quality. This is due to the mismatch of the aspherical correction and the optical properties of the eye model. Therefore it is recommended to implant the IOL in accordance with the guidelines given by the manufacturer. It can be concluded that if a reversed aspherical IOL causes degradation of the optical performance of the entire optical pathway it introduces a mismatch in the optical path lengths of the imaging system. The image forming converging wavefront is aberrated and deviates from the spherical shape. Since the entire optical pathway consists of both corneal surfaces, both IOL surfaces and the embedding media the aberration balance of the surface contributions is not achieved. The important question of how the corneal topography needs to be shaped to restore a balanced aberration free optical system leads to a better understanding of the scope of the invention. Optical calculation reveals the theoretical shape of the cornea for which the optical system with reversed aspherical IOL is in balance with zero amount of spherical aberration. In other words: For an asymmetrical aspherical IOL based on average corneal topography, if implanted in the reverse way, exists a non-average corneal shape for which the optical system is again well corrected for spherical aberration.

This conclusion is further expanded within the scope of the invention to the question of how an aspherical IOL must be shaped to serve for example an average population corneal topography in one direction of implantation and another well defined group of corneal shapes e.g. the average after receiving a myopic LASIK. This invention teaches how an asymmetrical aspherical IOL must be designed to serve to distinct groups of corneal topographies by selecting the direction of implantation upon surgery.

It is clinically quite difficult to cancel positive SA in all patients, because their ocular geometry varies and differs from what's present in eye models. It is also difficult to fully preserve corneal SA ('Aberration free strategy') in all cases for the same reasons. Therefore, this reversible IOL would be showing two tendencies: one orientation is close to aberration neutral, the other leads to some positive SA compensation.

In another aspect the IOL would have an orientation aimed at optimizing optical quality in post myopic surgery eyes (flat corneas, oblate asphericity). The opposite orientation is for post hyperopic surgery eyes (steep corneas, hyperprolate asphericity). This scenario would also be interesting in centered keratoconus corneas.

A novel aspherical IOL is designed in order to provide two distinct (and well defined) amounts of spherical aberration depending on the direction of implantation. The IOL is designed symmetrically with respect to the haptics centre to avoid shift in the ELP (effective lens position) depending on the direction of implantation. In order to support that, the haptics are angulated less than 5° preferably at zero degrees.

The Liou-Brennan eye model is modified and the lens according to the present invention will satisfy an improved visual acuity and contrast vision performance for two different scenarios, just by reversing the orientation. Doing this so, it's possible to have one lens which is able to correct different groups of eye-biometry beside the Liou-Brennan eye model or in combination with the averaged biometry, for example. One task applying the present invention could be a lens, which will have best visual performance for the average european cornea and, using the reversed orientation, best visual performance for the asian cornea. This will avoid two different types of lenses for both markets and, hence, afford for the lens manufacturer and will provide a nearly perfect solution for a broad group of both ethnic groups.

Another task applying the present invention could be a lens, which is corrected for typical biometry of eyes treated by refractive surgery. The first orientation of the lens could be used to correct spherical aberration of the averaged Liou-Brennan eye. The second orientation of the same lens will correct the typical spherical aberration of post refractive surgery treated myopic or hyperopic eyes. In the same sense, it's possible to correct eyes with biometry of typical post LASIK surgery treated eyes for myopic and hyperopic correction due to change the orientation of the same lens. This kind of lens would provide much better optical performance for foresaid groups of patients, which would have very poor visual performance with an IOL designed by prior art methods.

Another task applying the present invention could be a lens, which is corrected for different radii of the cornea, to provide a good visual performance for patient beside the averaged cornea model. Again one orientation could be used to correct very steep cornea shapes and the other orientation could be used to correct very flat cornea shapes. This kind of IOL would lead to more customized IOL implantation with benefit on optical performance for groups of patients beside of averaged corneas.

Of course there are manifold variations applicable to the present invention, apart from the description above. The basic scope of the invention is to have one special lens in the field of ophthalmology, which is designed purposeful to deal two different correction tasks, depending on its orientation.

An aspherical shape that allows the aforementioned optical performance and capabilities can be described by the equation:

$$z = \frac{cr^2}{1+\sqrt{1-(1+Q)c^2 r^2}} + k_2 r^2 + k_4 r^4 + k_6 r^6 + k_8 r^8$$

with     (curvature = 1 / base radius of curvature)

r =      independent variable, radius about optical axis

Q =      conic constant

$k_n$ =      polynomial coefficient of order n

Rotational symmetric polynomial aspheric surfaces are described by a polynomial expansion of the deviation from a spherical (or aspheric described by a conic) surface. The even aspherical surface model uses only the even powers of the radial coordinate to describe the asphericity. The model includes the base radius of curvature and the conic constant.

The coefficients of the polynomial expansion, as well as the base radius are determined numerically in order to satisfy a least square fit to a particular merit function. This merit function accounts for the surgical statistics as described above and is minimized for optical performance.

An example of a possible layout of a modelled and manufactured intraocular lens 14 can be seen in Fig. 2. The lens 14 consists of an optics part 15 with at least two refracting surfaces 16 and 17. At least one of the refracting surfaces 16 and 17 is made of an aspherical shape profile. The lens can be placed and centered either in the empty capsular bag or the sulcus using the haptics portion 18. The haptics design can be a plate haptic design as widely described in the prior art or variations of the C-loop concept. Any variations in the haptics concept are widely known from prior art and are subsumed within the scope of this invention.

The lens can be made of soft and foldable hydrophilic or hydrophobic acrylates, hydrophilic acrylates with surface modifications such as hydrophobization, hydrogels, silicon, collamers or any other soft and transparent biomaterial suitable for IOL implants.

The single lens 14 is modelled in such a way that at least two different visual defects are corrected at least partially. One visual defect is corrected if the lens 14 is positioned in an eye with a first orientation and a second visual defect is corrected if the lens 14 is positioned in an eye with a second orientation. When positioned with the first orientation the surface 16 is turned towards the cornea of the eye and is therefore nearer to the cornea as the surface 17 and is than the anterior surface of the lens 14. When positioned with the second orientation the surface 17 is turned towards the cornea of the eye and is therefore nearer to the cornea as the surface 16 and than the surface 17 is the anterior surface.

When modelling the lens 14 preferably a computer programme is used. Modelling the lens 14 is an iterative process. First the two differet visual defetcs to be corrected with lens 14 are defined. Than two mathematical models are chosen and changed in such a way that the both visual defetcs are defined by the models. In the embodiment of the invention the Liou Brennan model is chosen with its basic state and for defining the visual defetcs to be corrected with lens 14 to be modelled in the modelling process, two different specifications of said basic state are created and based. Therefore two different configurations are defined. Then in a further step connections or links of parameters of these configurations are created. The connection of the parameters is defined in such a way that changing a parameter in one configuration the connected parameter in the other configuration is changed as well, especially changed in the same way. Therefore if the shape of the anterior surface of the lens in the first configuration is changed the shape of the anterior surface of the second configuration is changed as well. The anterior surface of the second configuration is the posterior surface of the first configuration and the anterior surface of the first configuration is the posterior surface of the second configuration. Further a merit function is defined and based evaluating if the result of the modelling process fulfills the defined correction limits. If this is not fulfilled in an acceptable manner the process goes on and before parameters of the lens are changed in one configuration. Because of the connection with the parameters in the other configuration these parameters of the other configurations are then changed automatically. The iterative process runs till both visual defects to be corrected are correctable with Strehl ratios each more than 0.95 with respect to the specifications of the different models based.

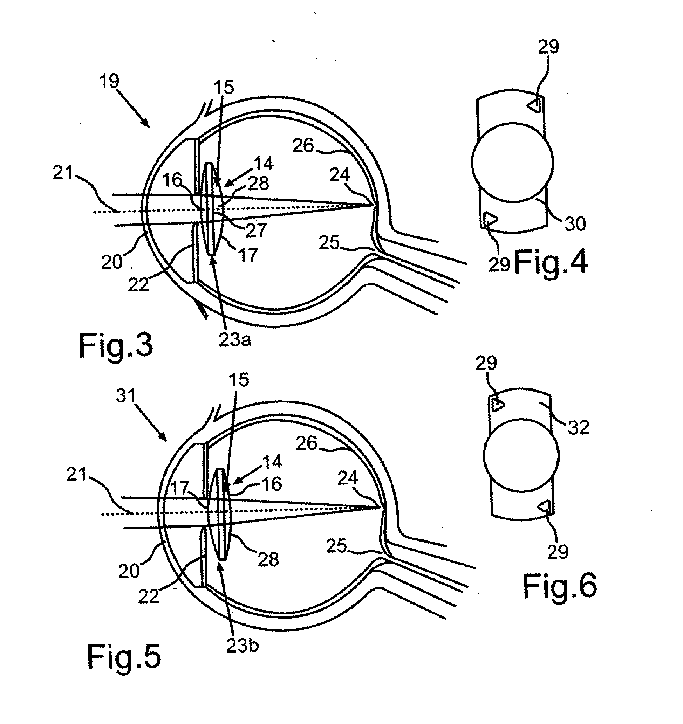

In Fig. 3 a sectional cut of a human eye 19 is shown. In the schematic drawing the lens 14 is implanted in the eye 19 in a first position with a first orientation. In said first orientation the surface 16 is turned towards the cornea 20. With this orientation of the lens 14 in the eye 19 a specific visual defect of the eye 19 which corresponds to the first visual defect to be correctable by th lens 14 is at least partially correctabe, especially with a Strehl ratio more than 0.95. The visual axis of the eye is given by 21. Further the iris 22, the macular region 24, the optical nerve head 25 and the retina 26 are shown. The aspherical IOL 14 is oriented with a direction marker 27 towards the cornea 20. In the described embodiment only the shape of the surfaces 16 and 17 are changed in the modelling process to get a lens 14 which is then manufactured based on the modelled lens. Therefore the interior 28 of the lens 14 has no gradient index. It is also possibe that instead of changing the surfaces 16 and 17 or in addition thereto the interior 28 of the lens 14 is modelled with a gradient index. Further the lens 14 is in a first position 23a within the eye 19.

As seen from the surgeon's perspective the markers 29 indicate the implantation direction 30 which is demonstrated in Fig. 4.

Without altering the idea of the invention the haptics of the IOL 14, the capsular bag and the sulcus region are not shown for simplicity.

According to Fig. 5 the IOL 14 with an identical shape than in Fig. 2 and 3 can be preferably implanted in a different patient's eye 31 in reversed direction and therefore with a second orientation. With this second orientation in the eye 31 the surface 17 is turned towards to the cornea 20. With this orientation a second visual defect is corrected, especially also with a Strehl ratio more than 0.95. The lens 14 is in a second position 23b within the eye 31.

As seen from the surgeon's perspective in Fig. 6 the markers 29 indicate the reversed implantation direction 32.

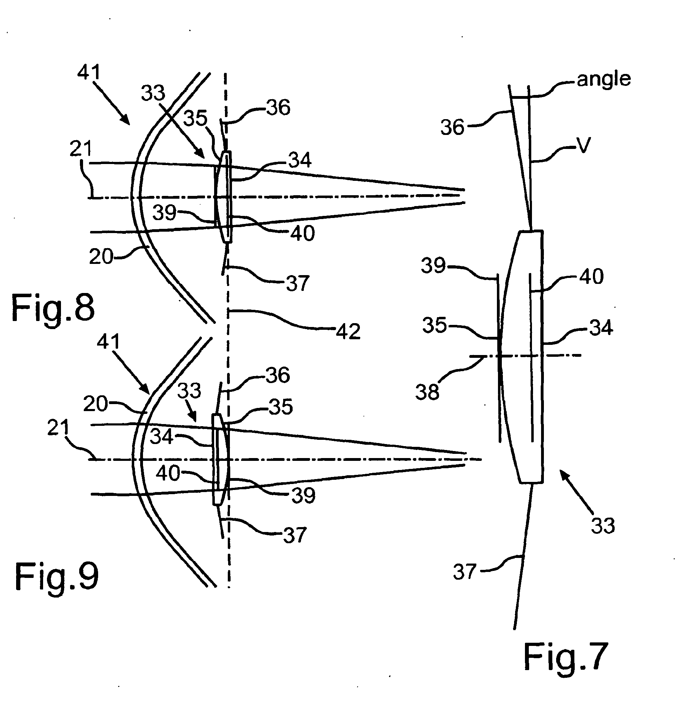

In Fig. 7 a sectional cut of a further embodiment of a lens 33 is shown. The lens 33 enables correcting at least two different visual defects depending on its orientation within a human eye. This embodiment of the lens 33 comprising a plane surface 34 and a aspherical surface 35. Haptic parts 36 an 37 are angled with respect to the vertical line V which is the lens equator plane. Especially the angulation of the haptic parts 36 and 37 is directed towards the aspherical surface 35. The angle is smaller than 10°. In Fig. 7 also the optical axis 38 and both principal planes 39 and 40 of the lens 33 are illustrated.

In Fig. 8, which illustrates a sectional cut, the lens 33 is implanted with a first position and with a first orientation in the eye 41. This means that the surface 35 is nearer to the cornea 20 than the surface 34. This defines a first orientation. Further because of angulation of the haptic parts 36 and 37 with this first orientation the lens 33 has a first position within the human eye 41 depending on the angulation of the haptic parts 36 and 37. As it can be seen in Fig. 8 with this first position the principal plane 40 coincides with the posterior principal plane location 42.

In Fig. 9, which illustrates a sectional cut, the lens 33 is implanted with a second position and with a second orientation in the eye 41. This means that the surface 34 is nearer to the cornea 20 than the surface 35. This defines a second orientation. Further because of angulation of the haptic parts 36, 37 with this second orientation the lens 33 has a second position within the human eye 41 depending on the angulation of the haptic parts 36 and 37. As it can be seen in Fig. 9 with this second position the principal plane 39 coincides with the posterior principal plane location 42.

With the angulation of the haptic parts 36 and 37 principal plane shift compensation is achieved.

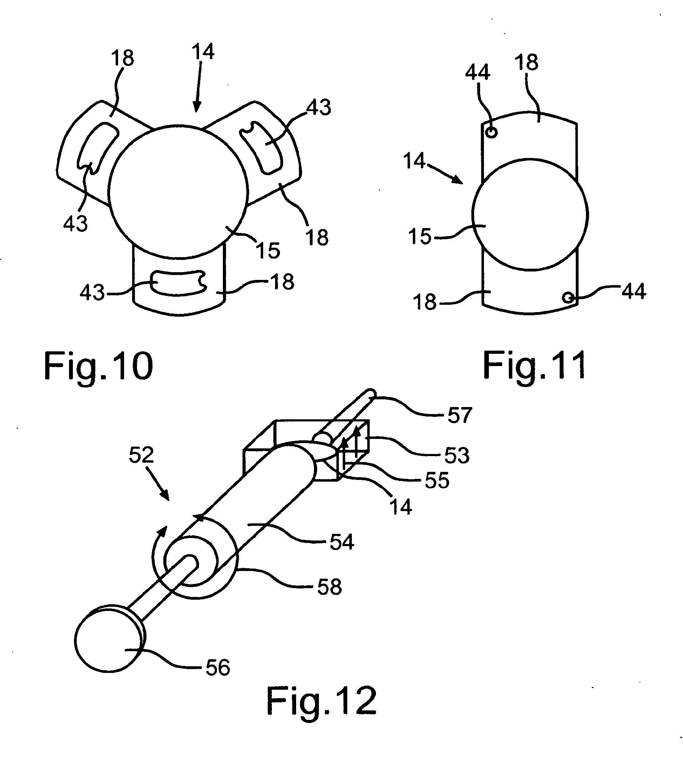

The IOLs 14 or 33 comprising means, e.g. markers that indicate the surgeon the orientation in the eye. Fig. 10 and 11 illustrate some examples of markers 43 and 44 that can be applied to single piece IOL 14. The same markers 43 or 44 are possible for the lens 33.

The reversion of the IOL 33 may cause the posterior principal plane to shift slightly with respect to the retinal image plane. A slight principal plane shift may change the overall power of the optical system and may lead to undesired refractive errors for the patient. This predictable shift is compensated by definition of two distinct A-constants depending on the direction of implantation. These A-constants are clearly indicated on a lens package or lens container.

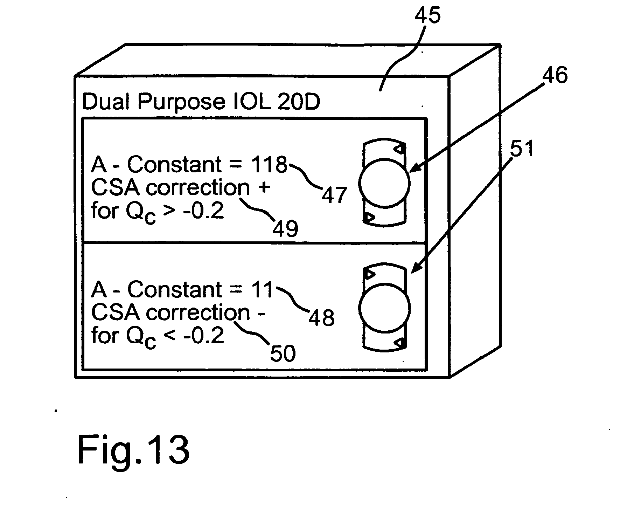

Fig. 13 shows an example of the dual purpose IOL lens container 45 wherein the lens is containable in a safe way an the lens can be shipped. The labeling part of the container 45 is split in at least two sections 46 and 51. These sections contain specific information for the two possible implantation options. E.g. the A-constant 47 and 48, a textual descriptor of the correction property 49 and 50 as well as an pictogram indicating the orientation markers as seen from the surgeons perspective during surgery.

In a different embodiment the principal plane shift due to the asymmetry of the IOL can be compensated by intentional slight angulation of the haptics. This angulation changes purposefully the effective position of the IOL in the eye. This change compensates for the principal plan shift. By use of this feature, the new IOL according to this invention can be labeled with just one A-constant instead of two.

In another embodiment the dual purpose IOL may be shipped as part of a pre-loaded injector or inserter system 52. Fig. 12 shows some major components of such a system 52. The lens 14 is contained in a lens storage chamber 53 of the injector system or in an insertable cartridge or cassette 53. The body of the lens container 53 or the injector body 54 comprising a marker 55 that indicates the orientation of the IOL 14 during injection. The injector system 52, comprising the plunger 56, the body 54, the container 53 and injection channel 57, is designed to provide repeatable ejection of the IOL 14 with predictable direction of rotation. Depending on the desired direction of implantation the injector may be rotated by 180° to support insertion of the IOL 14 in reversed order 58. The injection system 52 may be a single use preloaded system or a re-sterilizable system with cartridge port.

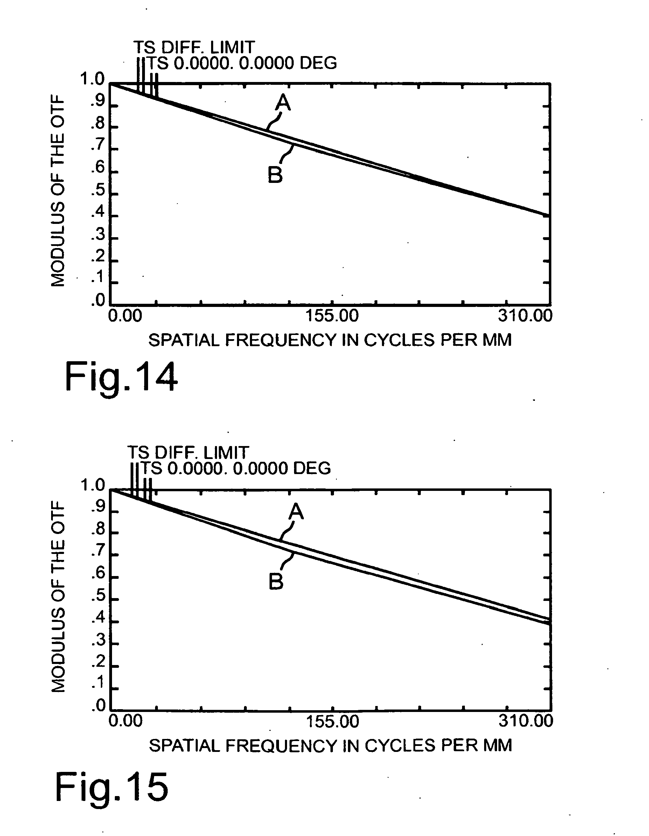

In the Fig. 14 to 21 some diagrams are illustrated where the modulus of the OTF (optical transfer function) depending of the spatial frequency in cycles is shown. The curves demonstrates the degrees of correction of visual defects with modelled lenses. To compare the quality of this correction the curve illustrating the theoretical refraction limit is shown as well.

The spatial frequency is a dimension for the pattern size. The larger the frequency the smaller the pattern. On the other side the modulus of the OTF is a dimension for the imaging quality and therefore a dimension for the achieved contrast.

In the Figs. 14 to 21 the curves A illustrates the physical limit. Therefore this illustrates the optimal imaging with respect of the based optical system. The diffraction limit of this system is characterized with a curve A. The curves B in Figs. 14 to 21 illustrates the imaging quality of the optical system, especially the model eye including the modelled intraocular lens. Therefore the tangential section and the sagittal section are provided. These sections provide the imaging quality of the system in the sheet plane and in a plane perpendicular thereto. Because of the fact that there is indicated only one axis bundle both curves are overlapped.

Therefore these diagrams disclose the imaging grade of the system at different pattern sizes. The curves indicate with which contrast the particular pattern size is imaged by the optical system. In this context a small structure or pattern, which is equal with a high frequeny, with a high contrast signify a good imaging quality.

In an embodiment an IOL is manufactured which will have best visual performance for the average european cornea and, using the reversed orientation, best visual performance for the asian cornea.

A further embodiment encompasses an IOL which is corrected for typical biometry of eyes treated by refractive surgery. The first orientation of the lens is used to correct spherical aberration of the averaged Liou-Brennan model eye. The second lens orientation corrects the typical spherical aberration of post refractive surgery treated myopic or hyperopic eyes.

In a further embodiment an IOL is manufactured which corrects eyes with biometry of typical post refractive surgery treated eyes for myopic and hyperopic correction due to change the orientation.

In a further embodiment an IOL is given which corrects eyes with different radii of the cornea. First orientation is used to correct very steep cornea shapes and the other orientation is used to correct very flat cornea shapes.

Preferably the reversible IOL showing two tendencies: one orientation is close to aberration neutral behavior, the other leads to some positive SA compensation.

Initial point for all variations of the eye models is the Liou-Brennan eye model.

Cornea shape, in particular the front surface, is the most important factor on generated optical performance, besides of the crystalline lens or IOL. Thus the cornea front surface is the object to change for simulation of several groups of typical biometry data. The corneal model assumptions will be described by the radius $R_{cornea}$ of the front surface of the cornea, and the conic constant $Q_{cornea}$ of the front surface of the cornea for each example. The radius of the back surface of the cornea will be changed by a fixed Gullstrand factor with respect to the front surface: $R_{cornea\ BACK} = R_{cornea} * 1.2140625$.

In order to get the best possible focus the length of the eye, in particular the length of the vitreous is adjusted. Best focus is optimized for each example to get contrast values (diffraction MTF) as high as possible for a range of spatial frequencies at the retina. Important presumption is the eye entrance pupil diameter, which was set to 6.0 mm. Contrast values will be shown over the range of spatial frequencies from 0 to 312 LP/mm at the retina to illustrate the optical performance in the given eye model.

In Figs. 14 and 15 an example is illustrated where an intraocular lens is modelled which, depending on its orientation in the eye, enables correcting an european cornea as defined as a first visual defect of a human eye and enables correcting an asian cornea as defined as a second visual defect of a human eye. One IOL which will have best visual performance for the averaged european cornea (represented by the Liou-Brennan eye model data with $R_{cornea}$=7,77 $Q_{cornea}$=-0,18) and, using the reversed orientation, best visual performance for the averaged asian cornea ($R_{cornea}$=7,63 $Q_{cornea}$=-0,114) is given.

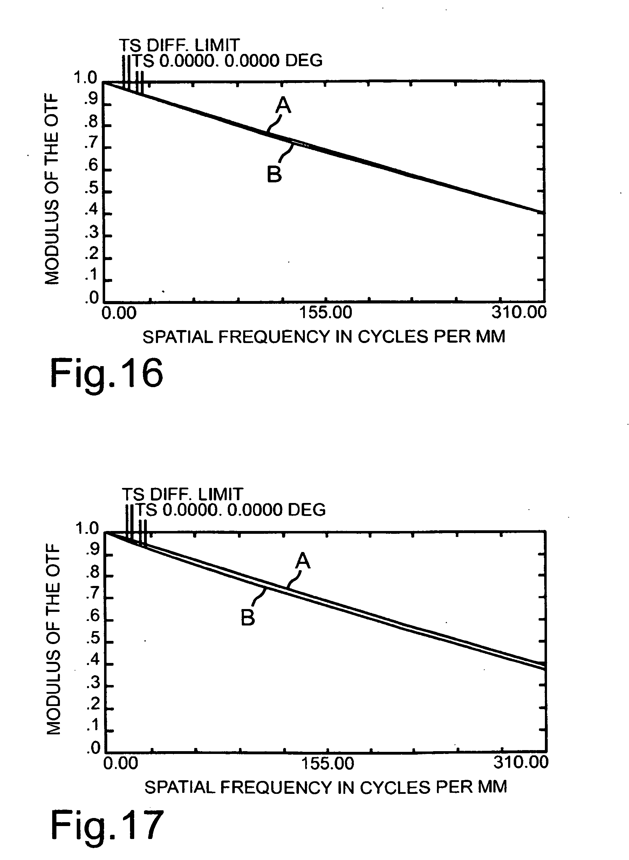

In a further example, which is illustrated in Figs. 16 and 17, an IOL is modelled and integrated in the system, which corrects eyes with typical biometry of typical post refractive surgery treated eyes. The first orientation of the lens used to correct spherical aberration of the averaged Liou-Brennan eye ($R_{cornea}$=7,77 $Q_{cornea}$=-0,18). With the second orientation of the lens it corrects the typical spherical aberration of post refractive surgery treated myopic ($R_{cornea}$=8,2 $Q_{cornea}$=+0,5) or hyperopic eyes ($R_{cornea}$=7,5 $Q_{cornea}$=-0,5). In Fig. 16 an orientation with averaged "normal" cornea is shown. In Fig. 17 an orienation typical cornea of post refractive surgery for a myopic eye is shown.

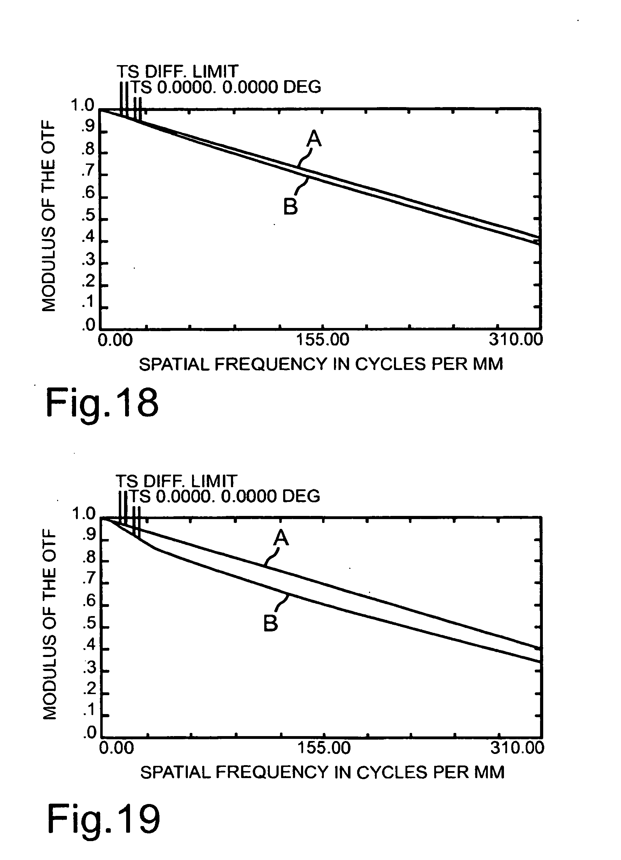

In Figs. 18 and 19 an IOL is modelled and integrated in the system, which corrects eyes with biometry of typical post refractive surgery treated eyes for myopic (Fig. 19) and hyperopic correction (Fig. 18) due to change the orientation.

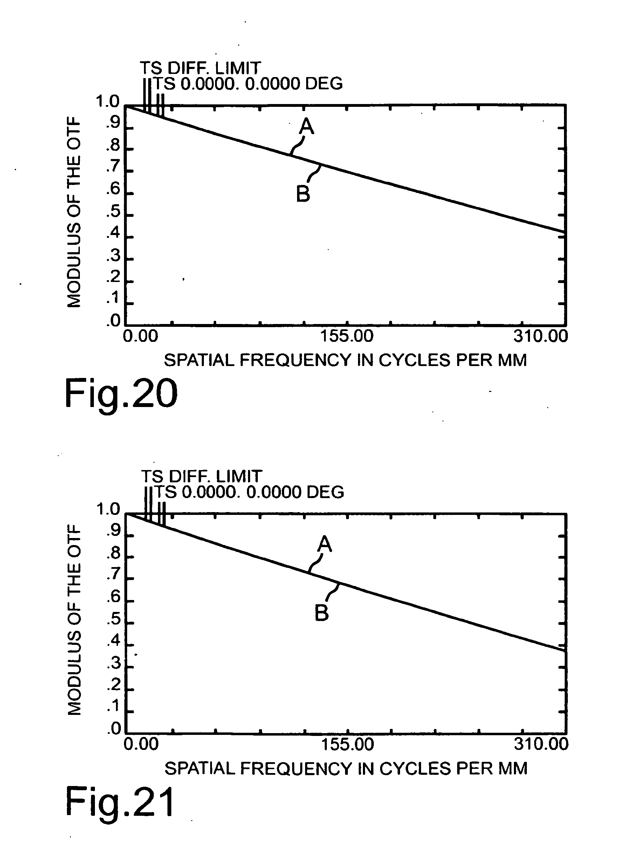

An IOL which corrects for the optical impact of different radii (keratometry) of the cornea is shown in Figs. 20 and 21. First orientation is used to correct very steep cornea shapes ($R_{cornea}$=7,4 $Q_{cornea}$=-0,18) (Fig. 20) and the other orientation is used to correct very flat cornea shapes ($R_{cornea}$=8,4 $Q_{cornea}$=-0,18) (Fig. 21).

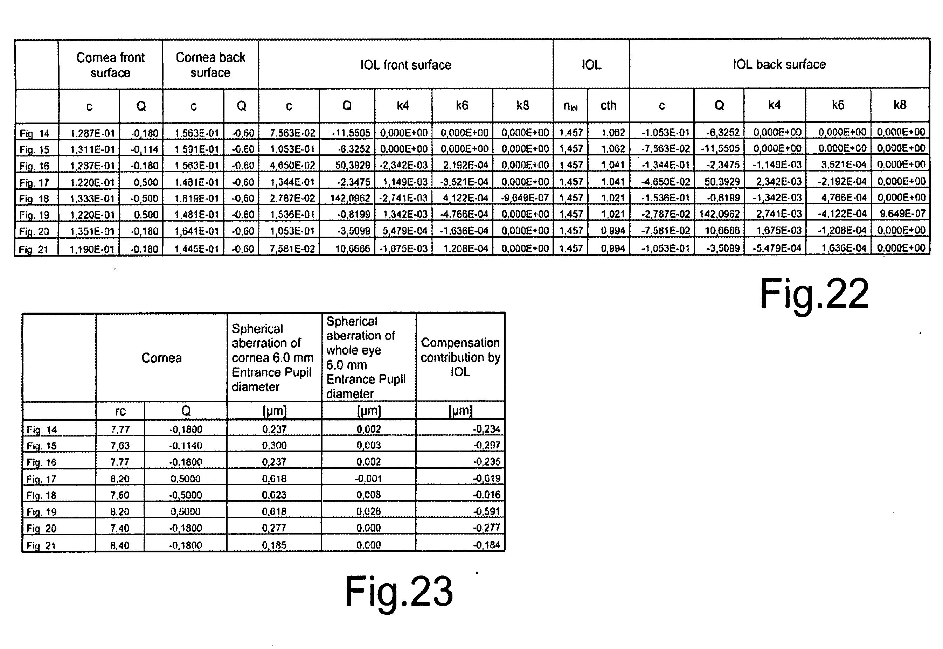

In Figs. 22 and 23 tables with design data (Fig. 22) and spherical aberration (Fig. 23) according to the examples in Fig. 14 to 21 are illustrated.

## Claims

1. A method for modelling a single intraocular lens, wherein the shape of at least one surface of the intraocular lens and/or the interior of the intraocular lens with respect to their refraction effect are configured in such a way that with a first orientation within a human eye the intraocular lens at least partially corrects a first visual defect of a human eye and with a second orientation within a human eye the intraocular lens at least partially corrects a second visual defect of a human eye, wherein the first orientation means that a first surface of the lens is turned towards the cornea when the lens is placed in a human eye, and the second orientation means that a second surface of the lens is turned towards the cornea, when the lens is placed in a human eye, and wherein said different visual defects are aberrations, and wherein at least one mathematical model of a human eye is used as a basis for modelling the intraocular lens by way of inserting the intraocular lens into said mathematical model to correct either said first or said second visual defect, depending on an orientation of the intraocular lens within a human eye, and wherein specific parameters of said mathematical model or said specification of a mathematical model used as a basis for characterizing the visual defects to be corrected by the intraocular lens are changed, and wherein the radius of the cornea and/or the curvature of the cornea and/or the conical constant for characterizing a specific visual defect are changed.

2. A method according to claim 1, wherein two different specifications of a mathematical model are used as a basis for modelling the intraocular lens for the correction of two different visual defects, wherein said first specification characterizes said first visual defect and said second specification characterizes said second visual defect.

3. A method according to claim 1, wherein two different mathematical models are used as a basis for modelling the intraocular lens in two different ways, wherein said first mathematical model characterizes said first visual defect and said second mathematical model characterizes said second visual defect.

4. A method according to one of the preceding claims, wherein said modelling generates an intraocular lens which functions as an aberration-correcting lens with a first orientation within a human eye.

5. A method according to one of the preceding claims, wherein said modelling generates an intraocular lens which functions as an aberration-corrected lens with a second orientation within a human eye.

6. A method according to claim 1, wherein said at least one mathematical model is **characterized by** an aspherical shape of the cornea and/or a gradient index of the refractive medium of said model eye and/or an aspherical shape of the natural human crystalline.

7. A method according to claim 1, wherein said mathematical model is the Liou- Brennan model.

8. A method according to claim 1, wherein the modelling includes defining the connections of at least one parameter characterizing the mathematical models or the specifications of a mathematical model used as a basis for modelling and in which upon changing the at least one parameter of one model or one specification one parameter of the other model or the other specification is changed in such a way that the shape of the surfaces of the intraocular lens and/or its interior with respect to its refraction effect is changed for correction of the first and the second visual defect.

9. A method according to one of the preceding claims, wherein at least one of said surfaces of the intraocular lens is designed as an aspherical surface.

10. A method according to one of the preceding claims, wherein modelling the intraocular lens is an iterative process.

11. A method according to one of the preceding claims, wherein at least one of said surfaces of said intraocular lens and/or said interior of said intraocular lens with respect to their refraction effect are configured in such a way that said first and said second visual defect, respectively, are corrected each at a Strehl ratio of at least 0.5, especially of at least 0.95, relative to the mathematical model or specification of said mathematical model used as a basis.

**Patentansprüche**

1. Verfahren zum Modellieren einer einzelnen Intraokularlinse, wobei die Form von mindestens einer Oberfläche der Intraokularlinse und/oder des Inneren der Intraokularlinse in Bezug auf ihre Brechwirkung in einer solchen Weise konfiguriert werden, dass bei einer ersten Orientierung innerhalb eines menschlichen Auges die Intraokularlinse zumindest teilweise einen ersten Sehfehler eines menschlichen Auges korrigiert, und bei einer zweiten Orientierung innerhalb eines menschlichen Auges die Intraokularlinse zumindest teilweise einen zweiten Sehfehler eines menschlichen Auges korrigiert, wobei die erste Orientierung bedeutet, dass eine erste Oberfläche der Linse in Richtung der Hornhaut gewandt ist, wenn die Linse in einem menschlichen Auge angeordnet ist, und die zweite Orientierung bedeutet, dass eine zweite Oberfläche der Linse in Richtung der Hornhaut gewandt ist, wenn die Linse in einem menschlichen Auge angeordnet ist, und wobei die verschiedenen Sehfehler Aberrationen sind und wobei mindestens ein mathematisches Modell eines menschlichen Auges als Basis zum Modellieren der Intraokularlinse durch Einsetzen der Intraokularlinse in das mathematische Modell verwendet wird, um entweder den ersten oder den zweiten Sehfehler in Abhängigkeit von einer Orientierung der Intraokularlinse innerhalb eines menschlichen Auges zu korrigieren, und wobei spezifische Parameter des mathematischen Modells oder die Spezifikation eines mathematischen Modells, das als Basis zum Charakterisieren der durch die Intraokularlinse zu korrigierenden Sehfehler verwendet wird, geändert werden, und wobei der Radius der Hornhaut und/oder die Krümmung der Hornhaut und/ oder die konische Konstante zum Charakterisieren eines spezifischen Sehfehlers geändert werden.

2. Verfahren nach Anspruch 1, wobei zwei verschiedene Spezifikationen eines mathematischen Modells als Basis zum Modellieren der Intraokularlinse für die Korrektur von zwei verschiedenen Sehfehlern verwendet werden, wobei die erste Spezifikation den ersten Sehfehler charakterisiert und die zweite Spezifikation den zweiten Sehfehler charakterisiert.

3. Verfahren nach Anspruch 1, wobei zwei verschiedene mathematische Modelle als Basis zum Modellieren der Intraokularlinse in zwei verschiedenen Weisen verwendet werden, wobei das erste mathematische Modell den ersten Sehfehler charakterisiert und das zweite mathematische Modell den zweiten Sehfehler charakterisiert.

4. Verfahren nach einem der vorangehenden Ansprüche, wobei das Modellieren eine Intraokularlinse erzeugt, die bei einer ersten Orientierung innerhalb eines menschlichen Auges als aberrationskorrigierende Linse fungiert.

5. Verfahren nach einem der vorangehenden Ansprüche, wobei das Modellieren eine Intraokularlinse erzeugt, die bei einer zweiten Orientierung innerhalb eines menschlichen Auges als aberrationskorrigierte Linse fungiert.

6. Verfahren nach Anspruch 1, wobei das mindestens eine mathematische Modell durch eine asphärische Form der Hornhaut und/oder einen Gradientenindex des Brechungsmediums des Modellauges und/oder eine asphärische Form der natürlichen menschlichen Kristalllinse **gekennzeichnet** ist.

7. Verfahren nach Anspruch 1, wobei das mathematische Modell das Liou-Brennan-Modell ist.

8. Verfahren nach Anspruch 1, wobei das Modellieren das Definieren der Verbindungen von mindestens einem Parameter, der die mathematischen Modelle oder die Spezifikationen eines mathematischen Modells, das als Basis zum Modellieren verwendet wird, charakterisiert, umfasst, und wobei beim Ändern des mindestens einen Parameters von einem Modell oder einer Spezifikation ein Parameter des anderen Modells oder der anderen Spezifikation in einer solchen Weise geändert wird, dass die Form der Oberflächen der Intraokularlinse und/oder ihres Inneren in Bezug auf ihre Brechwirkung zur Korrektur des ersten und des zweiten Sehfehlers geändert wird.

9. Verfahren nach einem der vorangehenden Ansprüche, wobei mindestens eine der Oberflächen der Intraokularlinse als asphärische Oberfläche ausgelegt ist.

10. Verfahren nach einem der vorangehenden Ansprüche, wobei das Modellieren der Intraokularlinse ein iterativer Prozess ist.

11. Verfahren nach einem der vorangehenden Ansprüche, wobei mindestens eine der Oberflächen der Intraokularlinse und/oder das Innere der Intraokularlinse in Bezug auf ihre Brechwirkung in einer solchen Weise konfiguriert werden, dass der erste bzw. der zweite Sehfehler jeweils mit einem Strehl-Verhältnis von mindestens 0,5, insbesondere von mindestens 0,95, relativ zum mathematischen Modell oder zur Spezifikation des als Basis verwendeten mathematischen Modells korrigiert werden.

**Revendications**

1. Procédé destiné à modeler une lentille intraoculaire simple, dans lequel la forme d'au moins une surface de la lentille intraoculaire et / ou l'intérieur de la lentille intraoculaire par rapport à leur effet de réfraction sont configurés de telle sorte qu'avec une première orientation dans un oeil humain, la lentille intraoculaire corrige au moins partiellement une première déficience visuelle d'un oeil humain et qu'avec une seconde orientation dans un oeil humain, la lentille intraoculaire corrige au moins partiellement une seconde déficience visuelle d'un oeil humain, dans lequel la première orientation signifie qu'une première surface de la lentille est tournée vers la cornée quand la lentille est placée dans un oeil humain et la seconde orientation signifie qu'une seconde surface de la lentille est tournée vers la cornée quand la lentille est placée dans un oeil humain et dans lequel lesdites déficiences visuelles différentes sont des aberrations et dans lequel au moins un modèle mathématique d'un oeil humain est utilisé en tant que base pour modeler la lentille intraoculaire au moyen de l'insertion de la lentille intraoculaire dans ledit modèle mathématique, pour corriger soit ladite première, soit ladite seconde déficience visuelle, en fonction d'une orientation de la lentille intraoculaire dans un oeil humain et dans lequel les paramètres spécifiques dudit modèle mathématique ou ladite spécification d'un modèle mathématique, utilisé en tant que base pour caractériser les déficiences visuelles à corriger par la lentille intraoculaire, sont modifiés et dans lequel le rayon de la cornée et / ou la courbure de la cornée et / ou la constante conique, permettant de caractériser une déficience visuelle spécifique, sont modifiés.

2. Procédé suivant la revendication 1, dans lequel deux spécifications différentes d'un modèle mathématique sont utilisées en tant que base pour modeler la lentille intraoculaire pour la correction de deux déficiences visuelles différentes, dans lequel ladite première spécification caractérise ladite première déficience visuelle et ladite seconde spécification caractérise ladite seconde déficience visuelle.

3. Procédé suivant la revendication 1, dans lequel deux modèles mathématiques différents sont utilisés en tant que base pour modeler la lentille intraoculaire de deux manières différentes, dans lequel ledit premier modèle mathématique caractérise ladite première déficience visuelle et ledit second modèle mathématique caractérise ladite seconde déficience visuelle.

4. Procédé suivant l'une des revendications précédentes, dans lequel ledit modelage produit une lentille intraoculaire qui fonctionne en tant que lentille correctrice d'aberration avec une première orientation dans un oeil humain.

5. Procédé suivant l'une des revendications précédentes, dans lequel ledit modelage produit une lentille intraoculaire qui fonctionne en tant que lentille à aberration corrigée avec une seconde orientation dans un oeil humain.

6. Procédé suivant la revendication 1, dans lequel ledit au moins un modèle mathématique est **caractérisé par** une forme asphérique de la cornée et / ou un gradient d'indice du milieu de réfraction dudit oeil modèle et / ou une forme asphérique du cristallin humain naturel.

7. Procédé suivant la revendication 1, dans lequel ledit modèle mathématique est le modèle de Liou & Brennan.

8. Procédé suivant la revendication 1, dans lequel le modelage comporte l'opération consistant à définir les liaisons d'au moins un paramètre qui caractérise les modèles mathématiques ou les spécifications d'un modèle mathématique utilisé en tant que base de modelage et dans lequel, lors du changement du au moins un paramètre d'un modèle ou d'une spécification, un paramètre de l'autre modèle ou de l'autre spécification est modifié, de telle sorte que la forme des surfaces de la lentille intraoculaire et / ou son intérieur par rapport à son effet de réfraction est modifié en vue de la correction de la première et de la seconde déficience visuelle.

9. Procédé suivant l'une des revendications précédentes, dans lequel au moins l'une desdites surfaces de la lentille intraoculaire est conçue en tant que surface asphérique.

10. Procédé suivant l'une des revendications précédentes, dans lequel le modelage de la lentille intraoculaire est un processus itératif.

11. Procédé suivant l'une des revendications précédentes, dans lequel au moins l'une desdites surfaces de ladite lentille intraoculaire et / ou ledit intérieur de ladite lentille intraoculaire par rapport à leur effet de réfraction sont configurés de telle sorte que ladite première et ladite seconde déficience visuelle, respectivement, sont corrigées chacune suivant un Strehl ratio d'au moins 0,5, spécialement d'au moins 0,95, par rapport au modèle mathématique ou à la spécification dudit modèle mathématique, utilisé en tant que base.

Fig.1

Fig.2

Fig.3

Fig.4

Fig.5

Fig.6

41
33
35
21
36
34
39
40
20
37
42

**Fig.8**

41
33
36
20
35
21
34
40
39
37

**Fig.9**

36
angle
V
39
40
35
34
38
33
37

**Fig.7**

Fig.10

Fig.11

Fig.12

Fig.13

TS DIFF. LIMIT
TS 0.0000. 0.0000 DEG

MODULUS OF THE OTF

1.0
.9
.8
.7
.6
.5
.4
.3
.2
.1
.0

A

B

0.00                    155.00                    310.00

SPATIAL FREQUENCY IN CYCLES PER MM

# Fig.14

TS DIFF. LIMIT
TS 0.0000. 0.0000 DEG

MODULUS OF THE OTF

1.0
.9
.8
.7
.6
.5
.4
.3
.2
.1
.0

A

B

0.00                    155.00                    310.00

SPATIAL FREQUENCY IN CYCLES PER MM

# Fig.15

TS DIFF. LIMIT
TS 0.0000. 0.0000 DEG

1.0
.9
.8    A
.7
.6    B
.5
.4
.3
.2
.1
.0

MODULUS OF THE OTF

0.00                    155.00                    310.00
SPATIAL FREQUENCY IN CYCLES PER MM

Fig.16

TS DIFF. LIMIT
TS 0.0000. 0.0000 DEG

1.0
.9
.8    A
.7
.6
.5    B
.4
.3
.2
.1
.0

MODULUS OF THE OTF

0.00                    155.00                    310.00
SPATIAL FREQUENCY IN CYCLES PER MM

Fig.17

Fig.18

Fig.19

TS DIFF. LIMIT
TS 0.0000. 0.0000 DEG

MODULUS OF THE OTF

1.0
.9
.8
.7
.6
.5
.4
.3
.2
.1
.0

A

B

0.00                    155.00                   310.00

SPATIAL FREQUENCY IN CYCLES PER MM

# Fig.20

TS DIFF. LIMIT
TS 0.0000. 0.0000 DEG

MODULUS OF THE OTF

1.0
.9
.8
.7
.6
.5
.4
.3
.2
.1
.0

A

B

0.00                    155.00                   310.00

SPATIAL FREQUENCY IN CYCLES PER MM

# Fig.21

| | Cornea front surface | | Cornea back surface | | IOL front surface | | | | | IOL | | IOL back surface | | | | |
|---|---|---|---|---|---|---|---|---|---|---|---|---|---|---|---|---|---|
| | c | Q | c | Q | c | Q | k4 | k6 | k8 | $n_{iol}$ | cth | c | Q | k4 | k6 | k8 |
| Fig. 14 | 1.287E-01 | -0,180 | 1.563E-01 | -0.60 | 7.563E-02 | -11,5505 | 0,000E+00 | 0,000E+00 | 0,000E+00 | 1.457 | 1.062 | -1.053E-01 | -6,3252 | 0,000E+00 | 0,000E+00 | 0,000E+00 |
| Fig. 15 | 1.311E-01 | -0,114 | 1.591E-01 | -0.60 | 1,053E-01 | -6,3252 | 0,000E+00 | 0,000E+00 | 0,000E+00 | 1,457 | 1.062 | -7.563E-02 | -11,5505 | 0,000E+00 | 0.000E+00 | 0,000E+00 |
| Fig. 16 | 1.287E-01 | -0.180 | 1.563E-01 | -0.60 | 4,650E-02 | 50,3929 | -2,342E-03 | 2.192E-04 | 0,000E+00 | 1.457 | 1.041 | -1.344E-01 | -2,3475 | -1,149E-03 | 3,521E-04 | 0.000E+00 |
| Fig. 17 | 1.220E-01 | 0,500 | 1.481E-01 | -0.60 | 1.344E-01 | -2.3475 | 1,149E-03 | -3,521E-04 | 0,000E+00 | 1.457 | 1.041 | -4.650E-02 | 50.3929 | 2,342E-03 | -2,192E-04 | 0,000E+00 |
| Fig. 18 | 1.333E-01 | -0.500 | 1.619E-01 | -0.60 | 2.787E-02 | 142,0962 | -2,741E-03 | 4,122E-04 | -9,649E-07 | 1.457 | 1.021 | -1.536E-01 | -0,8199 | -1,342E-03 | 4,766E-04 | 0,000E+00 |
| Fig. 19 | 1.220E-01 | 0.500 | 1.481E-01 | -0.60 | 1.536E-01 | -0,8199 | 1,342E-03 | -4,766E-04 | 0,000E+00 | 1.457 | 1,021 | -2.787E-02 | 142,0962 | 2,741E-03 | -4.122E-04 | 9.649E-07 |
| Fig. 20 | 1.351E-01 | -0,180 | 1.641E-01 | -0.60 | 1.053E-01 | -3,5099 | 5,479E-04 | -1,636E-04 | 0,000E+00 | 1.457 | 0.994 | -7.581E-02 | 10,6666 | 1,675E-03 | -1,208E-04 | 0.000E+00 |
| Fig. 21 | 1,190E-01 | -0.180 | 1.445E-01 | -0.60 | 7,581E-02 | 10,6666 | -1,075E-03 | 1.208E-04 | 0,000E+00 | 1.457 | 0,994 | -1.053E-01 | -3,5099 | -5,479E-04 | 1,636E-04 | 0,000E+00 |

# Fig.22

| | Cornea | | Spherical aberration of cornea 6.0 mm Entrance Pupil diameter | Spherical aberration of whole eye 6.0 mm Entrance Pupil diameter | Compensation contribution by IOL |
|---|---|---|---|---|---|
| | rc | Q | [µm] | [µm] | [µm] |
| Fig. 14 | 7.77 | -0,1800 | 0.237 | 0,002 | -0,234 |
| Fig. 15 | 7,63 | -0,1140 | 0.300 | 0,003 | -0,297 |
| Fig. 16 | 7.77 | -0,1800 | 0.237 | 0.002 | -0,235 |
| Fig. 17 | 8.20 | 0.5000 | 0,618 | -0.001 | -0,619 |
| Fig. 18 | 7.50 | -0,5000 | 0.023 | 0,008 | -0,016 |
| Fig. 19 | 8.20 | 0,5000 | 0,818 | 0,026 | -0,591 |
| Fig. 20 | 7.40 | -0,1800 | 0,277 | 0.000 | -0,277 |
| Fig. 21 | 8.40 | -0,1800 | 0,185 | 0,000 | -0,184 |

# Fig.23

EP 2 177 179 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6609793 B2

- DE 102006021521 A1

**Non-patent literature cited in the description**

- **R. Rawer.** Imaging quality of intraocular lenses. *Journal Cataract Refractive Surgery,* August 2005, vol. 31

- **Spraul ; Rawer.** Spielt die Orientierung der IOL im Auge eine Rolle. *Klin. Monatsblätter d. Augenheilkunde,* 2005, vol. 222, 972-976